(19) Europäisches Patentamt
European Patent Office
Office européen des brevets

(11) Veröffentlichungsnummer: **0 265 810**
**A2**

# (12) EUROPÄISCHE PATENTANMELDUNG

(21) Anmeldenummer: **87115302.9**

(22) Anmeldetag: **20.10.87**

(51) Int. Cl.4: **C12P 1/00**, C12P 17/06, C12N 5/02, //A61K35/78,A61K31/35

(30) Priorität: **25.10.86 DE 3636397**

(43) Veröffentlichungstag der Anmeldung: **04.05.88 Patentblatt 88/18**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Reinhard, Ernst, Prof. Dr.**
**Eckhofweg 7**
**D-7400 Tübingen(DE)**
Erfinder: **Mersinger, Rainer**
**Schwabstrasse 81**
**D-7400 Tübingen(DE)**
Erfinder: **Mandler, Andrea**
**Philosophenweg 81**
**D-7400 Tübingen(DE)**

## (54) Verfahren zur Gewinnung von Diterpenen vom Labdan-Typ, insbesondere von Forskolin, aus Coleus forskohlii.

(57) Die vorliegende Erfindung betrifft ein Verfahren zur Gewinnung von Diterpenen vom Labdan-Typ, insbesondere von Forskolin, aus Zellkulturen von Coleus forskohlii. Das Verfahren ist dadurch gekennzeichnet, daß die Zellkulturen von einem Erhaltungsmedium in ein Induktionsmedium übergeführt werden, das sich insbesondere hinsichtlich des Phytohormongehaltes vom Erhaltungsmedium unterscheidet, und aus ihnen nach einer bestimmten Zeitdauer die Diterpene durch Extraktion mit organischen Lösungsmitteln gewonnen werden.

EP 0 265 810 A2

Xerox Copy Centre

# Verfahren zur Gewinnung von Diterpenen vom Labdan-Typ, insbesondere von Forskolin, aus Coleus forskohlii

Die in Indien beheimatete Pflanze Coleus forskohlii Briq. aus der Familie der Lamiaceae enthält mehrere Diterpene vom Labdan-Typ. Mengenmäßig am stärksten vertreten sind 1,9-Didesoxy-forskolin (DDF) und Forskolin (BHAT et al., Tetrahedron Letters 19(1977) 1669-1672). Der Gehalt an Forskolin bezogen auf das Trockengewicht beträgt für die ganze Pflanze ca. 0,05 % und für die Wurzel ca. 0,1 % (SHAH et al., Planta Medica 39 (1980), 183-185).

Forskolin (= 7$\beta$-Acetoxy-8,13-epoxy-1$\alpha$,6$\beta$,9$\alpha$-trihydroxy-labd-14-en-11-on) hat die folgende Strukturformel:

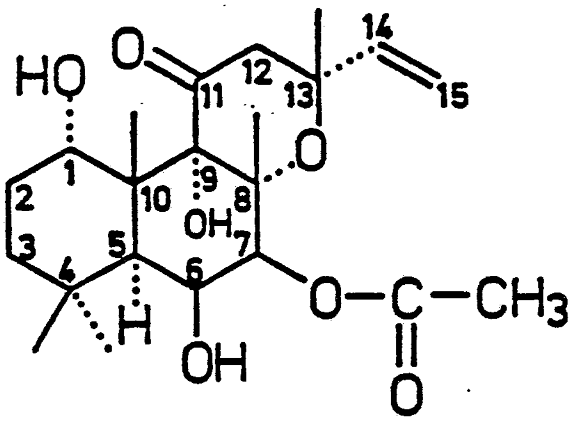

Entscheidend für das Interesse an der Pflanze Coleus forskohlii sind die ausgeprägten pharmakologischen Effekte des Forskolins. Es wirkt positiv inotrop, Herzfrequenz steigernd und Blutdruck senkend (LINDNER et al., Arzneim.-Forsch./Drug Research 28 (I) Heft 2 (1978), 284-289). Diese und zahlreiche andere Wirkungen werden vermittelt durch eine Aktivierung der membranständigen Adenylatcyclase, die zu einem Konzentrationsanstieg des intrazellulären cyclischen Adenosinmonophosphats (cAMP) führt (METZGER H., LINDNER E., Arnzneim.-Forsch./Drug Research 31 (II), Heft 8 (1981) 1248-1250; SEAMON K.B., DALY J.W., J. Cycl. Nucl. Res. 7 (4) (1981), 201-224; DE SOUZA N. et al., Med. Res. Rev. 3 (1983), 201-219). So hat Forskolin eine zweifache Bedeutung: Zum einen als Arzneimittel, zum anderen als ausgezeichnetes Hilfsmittel zur Untersuchung von Hormonwirkungen, die über cAMP als "second messenger" ausgelöst werden.

Gewonnen wird Forskolin bisher hauptsächlich aus wildwachsenden, z.T. auch aus angebauten Beständen von Coleus forskohlii durch Extraktion der getrockneten Wurzeln.

Das im folgenden beschriebene Verfahren gemäß der vorliegenden Erfindung stellt nun eine neue Möglichkeit dar, die verschiedenen Diterpene, besonders aber Forskolin zu gwinnen.

Dazu werden Zellkulturen von Coleus forskohlii als Suspensionskulturen gehalten. Auf dem sogenannten Erhaltungsmedium kommt es zu keiner oder nur geringfügiger Bildung von Forskolinderivaten. Um die Produktion anzuregen, werden die Zellaggregate in ein "Induktionsmedium" überführt, das sich vor allem in Bezug auf die Phytohormon-Anteile vom Erhaltungsmedium unterscheidet. In einem Zeitraum von 2 -6 Wochen erreicht die Diterpenbildung ihr Maximum und es ergeben sich Konzentrationen von 0,005 - 0,06 % Forskolin bezogen auf getrocknetes Zellmaterial.

Der Vorteil dieser neuen Produktionsmethode ist die Unabhängigkeit der Substanzbereitstellung von jahreszeitlichen und klimatishen Faktoren; sie ist außerdem nicht an den Standort Indien gebunden und vermindert damit auch wirtschaftliche und politische Risiken.

Das erfindungsgemäße Verfahren wird im folgenden genauer beschrieben. Zunächst werden nach bekannten Methoden Kalluskulturen von Coleus forskohlii - Pflanzen angelegt. Nach einigen Passagen (= Kulturperioden) auf mit Agar verfestigtem Nährmedium können die Zellaggregate in flüssiges Medium überführt und dort als Suspensionskulturen erhalten werden.

Geeignete Kulturbedingungen für die Erhaltungskultur sind:

-Aufbewahrung in Glasgefäßen, z.B. Erlenmeyerkolben
-Belüftung durch Schütteln auf einer Rundschüttelmaschine mit 80 - 150 rpm (revolutions per minute)
-Temperatur 18 - 38°C
-Dauerlicht, Dauerdunkel oder Licht-Dunkel-Rhythmus

Als Erhaltungsmedien können übliche Pflanzenzellkultur-Medien verwendet werden, z.B. Gamborgs B5-Medium (GAMBORG, D.L., Plant Physiol. 45, 372 (1970)) oder MS-Medium (MURASHIGE, T. und SKOOG, F., Plant Physiol. 28, 473 - 497 (1962) unter Zusatz von Phytohormonen, z.B. Auxinen wie 2,4-Dichlorphenoxyessigsäure (2,4-D), Indol-3-buttersäure (IBA) oder Naphthylessigäure (NAA) in einer geeigneten Konzentration, z.B. von 0,2 - 5 mg/l, bevorzugt 0,5 -2 mg/l, und/oder Cytokininen wie Kinetin oder 6-Benzylaminopurin (BAP) in einer geeigneten Konzentration, z.B. von 0,05 mg - 2 mg/l, bevorzugt 0,1 - 0,5 mg/l.

Der entscheidende Schritt zur Anregung der Forskolinproduktion besteht darin, die Zellen in ein "Arbeits-oder Induktionsmedium" zu bringen. Dieses Medium unterscheidet sich vom Erhaltungsmedium dadurch, daß es entweder.

a) keine Phytohormone enthält, oder

b) an Stelle von stark entdiffernzierend wirkenden Auxinen wie z.B. 2,4-D ein oder mehrere andere Auxine, wie z.B. IBA oder NAA enthält, oder

c) keine Auxine, sondern nur Cytokinine enthält, oder

d)Auxine und/oder Cytokinine in niedrigeren Konzentrationen als im Erhaltungsmedium enthält.

Das Induktionsmedium wird in 7 - 30 tägigem Rhythmus erneuert und nach jeder Passage ein Teil des Zellmaterials zur Forskolingewinnung abgeerntet.

Während der Produktionsphase erfolgt die Kultivierung bevorzugt im Dauerdunkel.

In der ersten Induktionspassage wird zunächst ein noch kaum verändertes Kulturwachstum beobachtet und die Bildung von Forskolinderivaten beschränkt sich meist auf kleine Mengen an 1,9-Didesozyforskolin (DDF) und Spuren der anderen Diterpene. In der zweiten oder dritten Induktionspassage wird dann bereits der maximale Gehalt an Forskolin und seinen Derivaten erreicht. Es bildet sich das gesamte Diterpenspektrum, das sich auch in der Wurzel der Pflanze findet.

Die Fortführung der Produktionsphase ist bis zu einer Dauer von mehreren Monaten im Erlenmeyerkolben möglich. Allerdings sind Forskolingehalt und Kulturwachstum stark abhängig davon, ob auf hormonfreiem Medium (→Produktionsphase ca. 3 Passagen) oder ob in Gegenwart von Phytohormonen in Form einer Auxin/Cytokinin-Kombination gearbeitet wird. Beispielsweise ist ein Medium mit 1,0 mg/l IBA und 0,2 mg/l BAP für eine Langzeitproduction geeignet.

Zur Gewinnung von diterpenhaltigem Zellmaterial in größerem Maßstab wird die Kultur in handelsüblichen Fermentern auf dem Induktionsmedium kultiviert. Die Vorzucht (d.h. die Passage(n) vor Beginn eines Induktionsversuches) für einen solchen Versuch findet bevorzugt im Erlenmeyerkolben statt, kann jedoch auch in einen Fermenter verlegt werden, aus dem auf geeignete Weise das Erhaltungsmedium entfernt werden kann.

Nach Abschluß eines Produktionsversuches werden Zellen und Nährmedium getrennt und letzteres verworfen, da es nur geringfügige Mengen an Diterpenen enthält. Die Zellen werden getrocknet und analog den Wurzeln mit organischen Lösungsmitteln (Toluol, Dichlormethan, Methanol u.a.) extrahiert (siehe z.B. DE Patent Nr. 25 57 784). Aus dem Rohextrakt werden durch Chromatographie z.B. an Kieselgelsäulen - schließlich Forskolin und gegebenenfalls andere LabdanDerivate, z.B. 1,9-Didesoxyforskolin und 1-Desoxyforskolin isoliert.

Die quantitative Bestimmung erfolgt z.B. durch GLC, HPLC (INAMDAR et al., J. Pharm. Sci. 69 (1980), 1449-1451; Planta Medica 50 (1984), 30-34) oder DC-Scanning.

Beispiel 1

Induktion einer Licht-Suspension

Verwendet wird eine im Dauerlicht gehaltene Suspensionskultur von Coleus forskohlii.

Vorzucht

| | |
|---|---|
| Einwaage: | 16 g Zellmaterial (Frischgewicht) in 1 l-Erlenmeyerkolben mit 300 ml Medium |
| Medium: | B5, (Pflanzenzellkultur-Medium nach GAMBORG, Plant Physiol., 45 (1970), 372) mit 1,0 mg/l 2,4-D und 0,2 mg/l Kinetin |
| Temperatur: | 24°C |
| Schüttelfrequenz: | 100 rpm |
| Licht: | Dauerlicht |
| Passagedauer: | 14 Tage |
| Vermehrungsrate: | 12 |

Induktions- bzw. Produktionsphase

| | |
|---|---|
| Medium: | B5-Medium mit 0,4 mg/l IBA |
| Licht: | Dauerdunkel |
| Temperatur: | 24°C |
| Schüttelfrequenz: | 100 rpm |

weitere Angaben s. folgende Tabelle 1:

Tab. 1: Beispiel 1, Induktion einer Licht-Suspension (= Suspensionskultur, im Dauerlicht kultiviert) in drei aufeinanderfolgenden Passagen

| | 1. Indukt. Passage | 2. Indukt.-Passage | 3. Indukt.-Passage |
|---|---|---|---|
| Zellmaterialeinwaage (Frischgewicht pro 300 ml Medium) | 15 g | 16 g | 24 g |
| Passagedauer | 15 Tage | 13 Tage | 12 Tage |
| Vermehrungsrate * | 10,1 | 6,0 | 4,6 |
| Forskolin (% vom Trockengewicht) | (+) | 0,0516 | 0,0239 |
| DDF (% vom Trockengewicht | 0,0181 | 0,0542 | 0,0209 |

* Vervielfachungsfaktor der Zellmasse, bezogen auf gefriergetrocknete Zellen

Aufarbeitung und quantitative Bestimmung
-Gefriertrocknung der frischabgeernteten Zellen nach Abtrennen vom Nährmedium
-Pulverisierung des Zellmaterials im Porzellanmörser
-Extraktion der Diterpene mit Toluol (2 Stunden, 50°C) unter Drehen am - nicht evakuierten - Rotationsverdampfer
-Filtrieren und Einengen des Filtrats am Rotationsverdampfer zur Trockene (T= 50°C)
-Aufnahme des Rohextraktes in Methanol im Ultraschallbad

Die quantitative Bestimmung der Forskolinderivate erfolgt nach dünnschichtchromatographischer Entwicklung und Detektion mittels DC-Scanning:

- Fließmittel = Toluol - Ethylacetat 85 : 15
- Entwicklung = 2 x 15 cm
- Platten = Kieselgel 60 $F_{254}$-Fertigplatten
- Detektion = Anisaldehyd/Schwefelsäure-Reagenz nach STAHL, Tauchbad; anschließendes Erhitzen bei 130-140°C, ca. 5 min
- Scanner = Fa. Desaga (Shimadzu Dual CS-930) Wellenlänge 500 nm

Beispiel 2

Fermentation mit Licht-Suspension, Produktion auf hormonfreiem Medium

Mit diesem Beispiel wird gezeigt, daß eine Kultivierung von Coleus forskohlii - Suspensionskulturen unter Induktionsbedingungen auch in größerem Maßstab möglich ist. Als Kulturbehältnis dient ein 20 1 - Airlift-Fermenter (WAHL J., Dissertation Tübingen 1977) mit der Möglichkeit, den Gehalt an gelöstem Sauerstoff im Medium ($pO_2$) automatisch konstant zu halten.

Zunächst wird im Fermenter eine Wachstumspassage auf normalem Erhaltungsmedium (vgl. BEISPIEL 1) durchgeführt, anschließend die Zellsuspension bis auf einen Rest von 4 l entnommen und der Fermenter mit hormonfreiem B5-Medium wieder auf das Arbeitsvolumen von 19 l aufgefüllt.

In der nun folgenden ersten Induktionspassage ist das Kulturwachstum weiterhin sehr stark; bereits nach etwa 13 Tagen ist ein Sinkvolumen (PCV) von 95 % erreicht. In den Zellen sind danach geringe Mengen an DDF, jedoch kein Forskolin nachweisbar.

Zur Fortführung des Versuchs wird erneut die Zellsuspension bis auf einen Rest von 3,5 l entnommen und durch frisches hormonfreies B5-Medium ersetzt. In dieser zweiten Induktionspassage setzt die Bildung von Diterpenen verstärkt ein und erreicht ihr Maximum von 0,0053 % Forskolin und 0,0116 % DDF am 9. Tag, der also den optimalen Zeitpunkt zur Zellmaterial-Aberntung darstellt. Bei weiterer Fortdauer der Fermentation verringert sich der Diterpengehalt stetig auf Grund eines teilweisen Absterbens der Kultur.

Daten zur Fermentation

| | |
|---|---|
| Kultur: | Licht-Suspension von Coleus forskohlii |
| Vorzucht in Kolben: | 15 Tage, Einwaage 16 - 17 g FG in 300 ml B5-Erhaltungsmedium (Frischgewicht= Gewicht der feuchten Zellen nach Ablaufen des Mediums) |
| Temperatur: | 24°C |
| Arbeitsvolumen: | 19 l |
| Saccharose/Glucose: | durch Zugabe von Saccharoselösung (30 %) wird ein Rest-Glucose-Wert > 0,5 % aufrechterhalten. |

Wachstumspassage (diffuses Tageslicht)

| | |
|---|---|
| Medium: | B5 (1,0 mg/l 2,4-D und 0,2 mg/l Kinetin) |
| Inokulum: (Animpfmenge) | 2,4 l = 8 Vorzuchtkolben |
| Dauer: | 16 Tage |
| $pO_2$: | 35 %, ab 14. Tag ansteigend bis 50 % |

1. Induktionspassage (Dauerdunkel)

| | |
|---|---|
| Medium: | B5, hormonfrei |
| Inokulum: | 4 l Suspension aus der Wachstumspassage |
| Dauer: | 13 Tage |
| $pO_2$: | 35 % |
| Antischaum: | Zugabe von insgesamt ca. 250 ml Polypropylenglykol-Lösung |

2. Induktionspassage (Dauerdunkel)

| | |
|---|---|
| Medium: | B5, hormonfrei |
| Inokulum: | 3,5 l Suspension aus der 1. Induktionspassage |
| Dauer: | 26 Tage |
| $pO_2$: | von 70 % auf 40 % absinkend |
| Belüftungsrate: | vvm= 0,06 - 0,10 (= 70 - 110 l Luft pro Stunde und pro 19 l Arbeitsvolumen) |

| Gehalt an Diterpenen (Gew.-%) | Forskolin | DDF |
|---|---|---|
| Tag 7 | 0,0035 | 0,0092 |
| Tag 8 | 0,0032 | 0,0093 |
| Tag 9 | 0,0053 | 0,0116 |
| Tag 11 | 0,0016 | 0,0102 |

Beispiel 3

Induktion einer Licht-Suspension, Isolierung von Diterpenen

Verwendet wird eine im Dauerlicht gehaltene Suspensionskultur von Coleus forskohlii.
Vorzucht: wie in Beispiel 1
Induktions-bzw. Produktionsphase: wie in Beispiel 1

davon abweichend

| | |
|---|---|
| Medium: | B5, hormonfrei |
| Einwaage: (2. Ind.-Passage) | 22,5 g und 29,5 g FG/300 ml Medium |
| Passagedauer. | 1. Induktionspassage 15 Tage<br>2. Induktionspassage 11 Tage |

Am Ende der zweiten Inkuktionspassage werden die Zellen zur Diterpen-Isolierung abgeerntet.

| | |
|---|---|
| Extraktion: | 2,26 g gefriergetrocknete Zellen mit Toluol (vgl. Beispiel 1) |
| Rohextrakt: | 68,6 mg = 3,0 % - in Methanol nur teilweise löslich |

Die Reindarstellung der Forskolinderivate wird durch säulenchromatographische Trennung erreicht.

| | |
|---|---|
| Säule: | Labomatic, ca. 50 ml, gefüllt mit Kieselgel 30 µm |
| mobile Phase: | Dichlormethan - Ethylacetat 8 : 1 |
| Extraktlösung: | Methanol wird als Lösungsmittel ersetzt durch die mobile Phase |
| zu trennende Substanzmenge: | ca. 25 mg (d.h. etwa 50 % des ursprünglichen löslichen Rohextraktes) |

Die gesamte eluierbare Substanzmenge von 12,8 mg ergibt u.a.:
0,7 mg Forskolin
1,0 mg 1,9-Didesoxy-forskolin (DDF)
0,4 mg 1-Desoxy-forskolin

Die Überprüfung der Identitäat der einzelnen Fraktionen erfolt durch GLC, HPLC (INAMDAR et al., 1980 und 1984) oder durch zweidimensionale Dünnschichtchromatographie.

| | |
|---|---|
| DC-Platten: | HPTLC, Kieselgel 60 $F_{254}$ 10 x 10 cm |
| Fließmittel: | I. Dichlormethan- Ethylacetat 8 : 1 |
| | II. Diisopropylether - Chloroform 7 : 3 |

**Ansprüche**

1. Verfahren zur Gewinnung von Diterpenen vom Labdan-Typ, dadurch gekennzeichnet, daß man Suspensionszellkulturen von Coleus forskohlii auf einem Erhaltungsmedium in Gegenwart von geeigneten Phytohormonen kultiviert, in den Zellkulturen dann durch Wechsel auf ein Nährmedium (Induktionsmedium), das sich vom Erhaltungsmedium hinsichtlich der Art und /oder der Konzentration der zugesetzten Phytohormone unterscheidet, die Diterpen-Bildung induziert und die Diterpene in an sich bekannter Weise isoliert.

2. Verfahren gemäß Anspruch 1, dadurch gekennzeichnet, daß als Erhaltungsmedium ein übliches Pflanzenzellkulturmedium unter Zusatz von Auxinen in einer Konzentration von 0,2 - 5 mg/l, und/oder Cytokininen in einer Konzentrationvon 0,05 - 2 mg/l, verwendet wird.

3. Verfahren gemäß Anspruch 2, dadurch gekennzeichnet, daß dem Erhaltungsmedium Auxine in einer Konzentration von 0,5 - 2 mg/l und/oder Cytokinine in einer Konzentration von 0,1 - 0,5 mg/l zugesetzt werden.

4. Verfahren gemäß Anspruch 1, dadurch gekennzeichnet, daß das Induktionsmedium sich vom Erhaltungsmedium dadurch unterscheidet, daß es
a) keine Phytohormone enthält, oder
b) an Stelle von stark entdifferenzierend wirkenden Auxinen wie 2,4-Dichlorphenoxyessigsäure (2,4-D) ein oder mehrere andere Auxine enthält, oder
c) keine Auxine, sondern nur ein oder mehrere Cytokinine enthält, oder
d) ein oder mehrere Auxine und/oder Cytokinine in niedrigerer Konzentration als im Erhaltungsmedium enthält.

5. Verfahren gemäß den Ansprüchen 1 bis 4, dadurch gekennzeichnet, daß im Erhaltungsmedium 2,4-Dichlorphenoxyessigsäure und/oder Naphthylessigsäure als Auxine verwendet und diese im Induktionsmedium durch Indol-3-buttersäure oder andere weniger entdifferenzierend wirkend Auxine ersetzt oder ersatzlos weggelassen werden.

6. Verfahren gemäß Anspruch 5, dadurch gekennzeichnet, daß das Induktionsmedium keine Phytohormone enthält.

7. Verfahren gemäß den Ansprüchen 1 bis 6, dadurch gekennzeichnet, daß mit vereinfachtem Medium (z.B. ohne Zusatz von Vitaminen, Aminosäuren) oder auch mit reinen Kohlenhydrat-Lösungen (z.B. Saccharose, Glucose) als Induktionsmedium gearbeitet wird.

8. Verfahren gemäß den Ansprüchen 1 bis 7, dadurch gekennzeichnet, daß Submerskulturen verwendet werden.

9. Verfahren gemäß den Ansprüchen 1 bis 8, dadurch gekennzeichnet, daß das Verfahren im Licht, im Dunkeln oder im Licht/Dunkel-Rhythmus durchgeführt wird.

10. Verfarhren gemäß den Ansprüchen 1 bis 9, dadurch gekennzeichnet, daß das Verfahren bei Temperaturen von 18 bis 38°C durchgeführt wird.

11. Verfahren gemäß den Ansprüchen 1 bis 9, dadurch gekennzeichnet, daß die Dauer einer Induktionsphase 7 bis 30 Tage beträgt.

12. Verfahren gemäß den Ansprüchen 1 bis 11, dadurch gekennzeichnet, daß mehrere Induktionsphasen hintereinander ablaufen, indem ein Teil der zur Diterpenproduktion angeregten Zellen jeweils auf frischem Induktionsmedium weiterkultiviert wird (semikontinuierliches Verfahren).

13. Verfahren gemäß den Ansprüchen 1 bis 12, dadurch gekennzeichnet, daß das Verfahren in Glasgefäßen, z.B. Erlenmeyerkolben (Belüftung durch Rundschütteln) oder in Fermentern (Belüftung durch Einleiten von Gasen) durchgeführt wird.

14. Verfahren gemäß den Ansprüchen 1 bis 13, dadurch gekennzeichnet, daß Forskolin gewonnen wird.